Europäisches Patentamt

European Patent Office

Office européen des brevets

(19)

(11) Veröffentlichungsnummer : **0 163 013**
**B1**

(12) **EUROPÄISCHE PATENTSCHRIFT**

(45) Veröffentlichungstag der Patentschrift :
04.05.88

(51) Int. Cl.⁴ : **A 61 F   2/36**

(21) Anmeldenummer : **85102389.5**

(22) Anmeldetag : **04.03.85**

(54) **Hüftprothesenschaft.**

(30) Priorität : **02.04.84 CH 1656/84**

(43) Veröffentlichungstag der Anmeldung :
**04.12.85 Patentblatt 85/49**

(45) Bekanntmachung des Hinweises auf die Patenterteilung : **04.05.88 Patentblatt 88/18**

(84) Benannte Vertragsstaaten :
**AT BE DE FR GB IT LU NL SE**

(56) Entgegenhaltungen :
EP-A- 0 112 423
DE-A- 2 331 728
DE-A- 2 805 305
DE-B- 2 324 865
DE-U- 8 213 101
GB-A- 2 118 441
US-A- 2 719 522

(73) Patentinhaber : **Emil Schenker AG**
**Stauwehrstrasse 34**
**CH-5012 Schönenwerd (CH)**

(72) Erfinder : **Karpf, Kurt**
**Alte Strasse 175**
**CH-4718 Holderbank (CH)**

(74) Vertreter : **Fillinger, Peter, Dr.**
**Rütistrasse 1a**
**CH-5400 Baden (CH)**

Jouve, 18, rue St-Denis, 75001 Paris, France

**Beschreibung**

Die vorliegende Erfindung bezieht sich auf einen Hüftprothesenschaft gemäss dem Oberbegriff des Anspruchs 1.

Ein derartiger Hüftprothesenschaft ist aus der EP-A-112423 (Stand der Technik gemäß Art. 54(3)) bzw. der DE-A-2805305 bekannt. Seine lateralen Schmalflächen sind gebogen, so dass sie unter Belastung eine Stützfunktion ausüben und ein Eindringen des Schaftes in den Femurknochen verhindern. In dieser Wirkung werden sie durch eine am oberen Schaftende angeordnete, auf dem Knochenrand liegende Schulter unterstützt. Bei einer zementlosen, reibschlüssigen Verankerung eines solchen Schaftes bestände die Gefahr, dass er sich unter der auftretenden Belastung lockert und in der Markhöhle verschiebt oder verdreht. Dieser Hüftprothesenschaft muss daher in die Markhöhle des Oberschenkelknochens einzementiert werden damit er die auf ihn wirkenden Kräfte auf den Oberschenkelknochen übertragen kann.

Die vorliegende Erfindung stellt sich die Aufgabe, einen Hüftprothesenschaft der eingangs erwähnten Art derart zu verbessern, dass bei reibschlüssiger Befestigung im Oberschenkelknochen ein Lockern unter den wechselnden Belastungen des Gehens ausgeschlossen wird und dass die Belastungskräfte auf möglichst grosse Flächen verteilt werden.

Erfindungsgemäss wird diese Aufgabe gelöst durch die kennzeichnenden Merkmale des Anspruchs 1.

Anhand der beiliegenden schematischen Zeichnung wird die Erfindung beispielsweise erläutert. Es zeigen :

Fig. 1 eine Vorderansicht eines Hüftprothesenschaftes,

Fig. 2 einen Schnitt längs der Linie II-II in Fig. 1,

Fig. 3 einen Schnitt längs der Linie III-III in Fig. 1 und

Fig. 4 einen Ausschnitt aus einem Hüftprothesenschaft in perspektivischer Darstellung, wobei die Proportionen gegenüber dem Beispiel nach den Fig. 1 bis 3 verzeichnet sind.

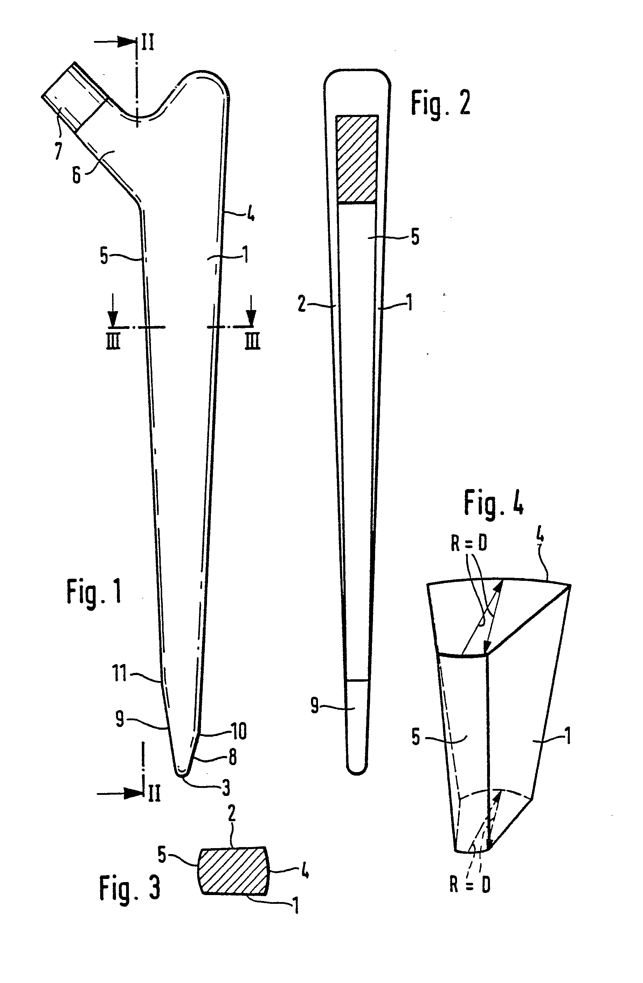

Der in Fig. 1 bis 3 gezeigte Hüftprothesenschaft weist eine vordere und hintere Breitfläche 1 bzw. 2 auf. Welche sich nach unten gegen einen gerundeten Schnitt 3 verjüngen. Weiter weist er eine laterale und eine mediale Schmalfläche 4 bzw. 5 auf, die sich ihrerseits von oben nach unten verjüngen. Am oberen Ende führt von der medialen Fläche ein Träger 6 schräg nach oben weg, der in einem Konus 7 endet, auf den eine Gelenkkugel aufsteckbar ist. Die Verjüngung der vier Flächen 1, 2, 4 und 5 erfolgt gegen einen gerundeten Spitz 3 am unteren Schaftende. Dadurch wirken die einander gegenüberliegenden Flächenpaare 1, 2 bzw. 4, 5 als Keilflächen, so dass bei entsprechender Vorbearbeitung der Knochenmarkhöhle sich der Schaft nach zwei Richtungen im Oberschenkelknochen festkeilt. Da sich Ungenauigkeiten beim Herausarbeiten der Auflageflächen im Knochen ergeben können, besteht die Gefahr, dass nur ein Keilflächenpaar gegenüber dem Knochen zum Tragen kommt und dass ein Spiel quer zur anderen Keilfläche entsteht. Um die Gefahr eines solchen Spiels zu beseitigen sind die im wesentlichen für die Kraftübertragung bestimmten Schmalflächen 4 und 5 konvex gewölbt. Der Wölbungsradius nimmt dabei von oben nach unten gegen die Schaftspitze hin ab und ist im wesentlichen gleich dem Schaftdurchmesser in der entsprechenden zur Schaftlängsachse rechtwinkligen Querschnittseben. Eine weitere Massnahme zur Verhinderung des genannten Spiels bildet das Konvergieren der beiden Breitflächen 1 und 2 gegen die mediale Schmalfläche (zusätzlich zur Konvergenz gegen die Spitze 3 wie dies aus den Fig. 2 bis 4 ersichtlich ist. Dabei wird vorzugsweise die mediale Schmalfläche schmäler ausgebildet als die laterale. Zur Vermeidung einer örtlichen Ueberlastung des Knochens ist weiter vorgesehen, die laterale und die mediale Schmalfläche 4 bzw. 5 gegen die gerundete Spitze 3 hin anzuschrägen. Die sich zwischen den Anschrägflächen 8 bzw. 9 und den entsprechenden Schmalflächen bildenden, gerundeten Schnittlinien 10 bzw. 11 sind in Richtung der Schaftlängsachse gegen einander höhenversetzt (Fig. 1). Dabei liegt die Schnittkante 10 mit der lateralen Schmalfläche 4 näher beim Spitz 3.

Die unterschiedlichen Anschrägungen 8, 9 an der Spitze des Schaftes verhindern eine sogenannte Ringspannung im Röhrenknochen. Die natürliche Elastizität des Knochens wird durch das unterschiedlich angeschrägte Schaftende nicht durch eine Spannungsspitze in einer zur Knochenlängsachse rechtwinkligen Ebene überbeansprucht. Frakturen durch Ueberbeanspruchung in diesem Bereich werden vermieden. Gleichzeitig dienen die beiden Anschrägungen auch zur besseren Einführung des Schaftes in die vorbereitete Markhöhle.

Um ein Abstützen des Schaftes im Kalkarbereich des Oberschenkels zu verhindern ist die Schmalflächen 4 geradlinig ausgebildet.

Die Rundung der Schmalflächen wirkt der Bildung von Spannungsspitzen im Knochen ebenfalls entgegen. Der von der lateralen und der medialen Schmalfläche 4 bzw. 5 eingeschlossene Winkel beträgt 5° bis 10° vorzugsweise 5°. Der von der lateralen Schmalfläche 4 und der angeschrägten Fläche 8 eingeschlossene Winkel beträgt vorzugsweise 165° bis 170°, wogegen der von der Anschrägung 9 und der medialen Schmalfläche 11 eingeschlossene Winkel vorzugsweise 170° bis 175° beträgt. Der von den Breitflächen 1 bzw. 2 eingeschlossene Winkel beträgt in einer zur Schaftlängsachse quer orientierten Ebene mindestens 4° und in einer Ansicht nach Fig. 2 zwischen 2° und 3° je Seite.

## Patentansprüche

1. Hüftprothesenschaft mit einer vorderen und hinteren Breitfläche (1, 2) sowie mit einer medialen und einer lateralen Schmalfläche (4, 5) und mit einem viereckigen Querschnitt, wobei jede der vier Flächen (1, 2, 4, 5) sich von oben nach unten gegen einen gerundeten Spitz (3) verjüngt, wobei am oberen Schaftende an der medialen Schmalfläche (5) seitlich ein Träger (6, 7) für eine Gelenkkugel wegragt und wobei von der medialen und lateralen Schmalfläche (4, 5) wenigstens eine konvex gewölbt ist und die Wölbungsradien (R) von oben nach unten stetig kleiner werden, dadurch gekennzeichnet, daß die einander gegenüberliegenden Flächen (1, 2 bzw. 4, 5) als Keilflächen wirken und dass die Wölbungsradien (R) in jeder zur Schaftachse rechtwinkligen Schnittebene jeweils näherungsweise gleich dem Abstand (D) der medialen und der lateralen Schmalflächen (4, 5) in dieser Schnittebene sind.

2. Hüftprothesenschaft nach Anspruch 1, dadurch gekennzeichnet, dass die mediale und die laterale Schmalfläche (4, 5) am unteren Ende eine gegen die Spitze (3) gerichtete Anschrägung (8, 9) aufweisen, dass die durch die Anschrägungen (8, 9) gebildeten Kanten (10, 11) von der Spitze (3) unterschiedlich entfernt sind, derart, dass die Entfernung auf der medialen Seite grösser als auf der lateralen Seite ist.

3. Hüftprothesenschaft nach Anspruch 1, dadurch gekennzeichnet, dass die Schmalflächen (4, 5) bis zu den Anschrägungen (8, 9) geradlinig verlaufen.

4. Hüftprothesenschaft nach Anspruch 1, dadurch gekennzeichnet, dass der Keilwinkel der Schmalflächen (4, 5) 5° bis 10° vorzugsweise 5° beträgt.

5. Hüftprothesenschaft nach Anspruch 1, dadurch gekennzeichnet, dass in Richtung der Schaftachse gesehen die beiden Breitflächen (1, 2) keilförmig konvergieren.

## Claims

1. Hip prosthesis stem with a front and rear wide surface (1, 2) and with a medial and a lateral narrow surface (4, 5) and with a square cross-section, each of the four surfaces (1, 2, 4, 5) tapering downwards to a rounded tip (3), a carrier (6, 7) for a joint head projecting laterally from the medial narrow surface (5) at the upper stem end and at least one of the medial and lateral narrow surfaces (4, 5) having a convex curvature and the radii (R) of curvature decreasing continuously downwards, characterized in that the mutually opposite surfaces (1, 2 and 4, 5) act as wedge surfaces and that the radii (R) of curvature in each section plane at right angles to the stem axis are always approximately equal to the distance (D) between the medial and lateral narrow surfaces (4, 5) in this section plane.

2. Hip prosthesis stem according to Claim 1, characterized in that the medial and lateral narrow surfaces (4, 5) have, at the lower end, a chamfer (8, 9) pointing towards the tip (3) and that the edges (10, 11) formed by the chamfers (8, 9) are at different distances from the tip (3), in such a way that the distance on the medial side is greater than that on the lateral side.

3. Hip prosthesis stem according to Claim 1, characterized in that the narrow surfaces (4, 5) extend in a straight line up to the chamfers (8, 9).

4. Hip prosthesis stem according to Claim 1, characterized in that the wedge angle of the narrow surfaces (4, 5) is 5° to 10°, preferably 5°.

5. Hip prosthesis stem according to Claim 1, characterized in that, as viewed in the direction of the stem axis, the two wide surfaces (1, 2) converge in the shape of a wedge.

## Revendications

1. Tige de prothèse de hanche, comportant une surface large antérieure et une surface large postérieure (1, 2) ainsi qu'une surface étroite latérale et une surface étroite interne (4, 5), et présentant une section transversale quadrangulaire, chacune des quatre surfaces (1, 2, 4, 5) allant en se rétrécissant de haut en bas vers une pointe arrondie (3), un support (6, 7), prévu pour une rotule, faisant saillie latéralement à l'extrémité supérieure de la tige sur la surface étroite interne (5), et l'une au moins des surfaces étroites latérale et interne (4, 5) étant bombée de façon convexe et les rayons de courbure (R) du bombement devenant constamment plus petits en allant du haut vers le bas, tige de prothèse de hanche caractérisée en ce que les surfaces situées à l'opposé l'une de l'autre (1, 2 ou 4, 5) agissent comme surfaces de coin, et en ce que les rayons de courbure (R) du bombement dans chaque plan de coupe se situant à angle droit par rapport à l'axe de la tige sont chaque fois approximativement égaux à la distance (D) entre les surfaces étroites latérale et interne (4, 5) dans ce plan de coupe.

2. Tige de prothèse de hanche selon la revendication 1, caractérisée en ce que la surface étroite interne et la surface étroite latérale (4, 5) comportent à leur extrémité inférieure un biseau (8, 9) dirigé vers la pointe (3), en ce que les arêtes (10, 11) formées par les biseaux (8, 9) sont éloignées à des distances différentes de la pointe (3) de telle manière, que l'éloignement est plus grand du côté de la surface interne que du côté de la surface latérale.

3. Tige de prothèse de hanche selon la revendication 1, caractérisée en ce que les surfaces étroites (4, 5) s'étendent en direction rectiligne jusqu'aux biseaux (8, 9).

4. Tige de prothèse de hanche selon la revendication 1, caractérisée en ce que l'angle de coin des surfaces étroites (4, 5) est de 5° à 10°, de préférence de 5°.

5. Tige de prothèse de hanche selon la revendication 1, caractérisée en ce que, vues dans la direction de l'axe de la tige, les deux surfaces larges (1, 2) convergent en forme de coin.

Fig. 1

Fig. 2

Fig. 3

Fig. 4

R = D

R = D